# NEW EUROPEAN PATENT SPECIFICATION

(11) **EP 1 287 048 B2**
(45) Date of publication and mention of the opposition decision: **30.04.2008**
(45) Mention of the grant of the patent: 27.04.2005
(21) Application number: 01960207.7
(22) Date of filing: 25.08.2001
(51) Int. Cl.: C08F 220/56, A61L 27/16, A61L 27/52, C08L 33/26

(54) **POLYACRYLAMIDE HYDROGEL AND ITS USE AS AN ENDOPROSTHESIS**
POLYACRYLAMID HYDROGEL UND SEINE VERWENDUNG ALS ENDOPROTHESE
HYDROGEL DE POLYACRYLAMIDE ET SON UTILISATION COMME ENDOPROTHESE

(30) Priority: 25.08.2000 DK 200001262
(43) Date of publication of application: 05.03.2003
(60) Divisional application: 04002645.2
(73) Proprietor: Contura S.A., 1820 Montreux (CH)
(72) Inventor: PETERSEN, Jens, DK-3460 Birkerod (DK); SCHMIDT, Richard, DK-2950 Vedbaek (DK); LESSEL, Robert, DK-2650 Brondby (DK); SORENSEN, Jens-Erik, DK-2900 Hellerup (DK)
(74) Representative: Plougmann & Vingtoft A/S
(86) International application number: PCT/DK2001/000565
(87) International publication number: WO 2002/016453

(56) References cited:
- EP-A- 0 727 232
- EP-A- 0 742 022
- WO-A-01/49336
- WO-A-99/10021
- RU-A- 2 127 129
- RU-A- 2 148 957
- US-A- 4 540 568
- DATABASE WPI Section Ch, Week 199602 Derwent Publications Ltd., London, GB; Class A14, AN 1996-018518 XP002154032 & RU 2 034 465 C (INTERFALL ENTERP), 10 May 1995 (1995-05-10)
- Ann. Plast. Surg., 2004 Sep. 53(3) pp. 267-272
- Aesthetic Plast. Surg. 202 Sep-Oct., 26(5), pp. 375-382

## Description

### FIELD OF INVENTION

The present invention relates to a novel polyacrylamide hydrogel cross-linked polyacrylamide. The hydrogel is obtainable by combining the acrylamide and methylene bis-acrylamide in a specific ratio so as to confer physical properties to the hydrogel. The present invention further relates to the use of the hydrogel for the preparation of an endoprosthesis for the treatment of incontinence, and for the treatment of vesicouretal reflux.

### BACKGROUND OF THE INVENTION

Natural and synthetic polymers such as collagen, soya, glycerol, silicone, polyvinylpyrolidone and hyaluronic acid have been utilised as endoprostheses. Materials used for endoprostheses generally try to imitate the natural soft tissue and are intended to be safe to the health of the patient.

Polyacrylamide gels have also been disclosed. US 5,798,096 relates to a biocompatible hydrogel containing 3.5 to 6.0% cross-linked polyacrylamide. However, US 5,798,096 teaches that concentrations below 3.5% make the hydrogel unstable.

GB 2114578 relates to a polyacrylamide gel for medical and biological purposes containing 3 to 28% polyacrylamide with the remainder of the mass of the gel comprised of a physiological solution.

US 5,658,329 relates to an implantable endoprosthesis comprising a shell filled with a polyacrylamide gel comprising 2 to 20% polyacrylamide by weight and a viscosity range of 15 to 75 Pas.

Formacryl® polyacrylamide is a soft-tissue endoprosthesis consisting of 5% reticulated polyacrylamide polymer and 95% apyrogenic water commercialised as an injectable device for medical and dental use to correct congenital or acquired deficits such as wrinkles, lines and scars. It is to be implanted with a syringue in the hypodermis.

US 5,306,404 relates to a process for preparing polyacrylamide gel plates for electrophoresis.

WO 99/10021 relates to an injectable, biocompatible hydrogel comprising 0.5 to 10% polyacrylamide and an antibiotic or antiseptic. WO 99/10021 is directed to the solving the problem of sappuration and rejection of the gel in its use an endoprosthesis.

### SUMMARY OF THE INVENTION

The generally invention relates to the biostable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution The bio-stable hydrogel typically has a molecular weight between 0.01 x 10⁶ and 20 x 10⁶. The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. The hydrogel is useful as an endoprosthetic amount, said gel tailored to the defect it seeks to correct.

The present invention relates in one aspect to a biocompatible hydrogel comprising i) less than 3.5 % (wt/wt) polyacrylamide cross-linked with methylene bis-acrylamide and ii) pyrogen-free water. Specifically, it relates to a hydrogel comprising less than 3.5% by weight polyacrylamide obtainable by combining acrylamide and methylene bis-acrylamide, radical initiation, and washing with pyrogen-free water, said combining being in a molar ratio of 150:1 to 1000:1.

An object of the invention is to provide a hydrogel for use as an injectable or implantable endoprosthesis comprising i) less than 3.5% (wt/wt) polyacrylamide cross-linked with methylene bis-acrylamide and ii) pyrogen-free water or saline solution.

An important aspect of the invention relates to a bio-stable hydrogel for use in the in the treatment and prevention of incontinence and vesicouretal reflux, said hydrogel obtainable by combining acrylamide and methylene bis-acrylamide in amounts so as to give 0.5 to 25% by weight polyacrylamide, based on the total weight of the hydrogel; radical initiation; and washing with pyrogen-free water or saline solution.

A further aspect of the invention relates to the use of a bio-stable hydrogel comprising 0.5 to 25% by weight polyacrylamide, based on the total weight of the hydrogel, for the preparation of an endoprosthesis for the treatment and prevention of incontinence and vesicouretal reflux and to a method of treating or preventing incontinence or vesicouretal reflux comprising administering a hydrogel to a mammal said hydrogel comprising 0.5 to 25% by weight polyacrylamide, based on the total weight of the hydrogel.

An important object of the invention is to provide a prosthetic device for increasing the resistance of conduits selected from the group consisting of the urethra; the rectum or colon; and the ureter for the treatment of urinary incontinence, anal incontinence, and vesicouretal reflux, respectively; wherein said device is injectable and comprising the bio-stable hydrogel of the invention.

### DETAILED DESCRIPTION OF THE INVENTION

### Hydrogels and Their Preparation

The success of plastic or reconstructive surgery depends to a great extent on the physical properties of the materials utilised. They must most certainly be biocompatible, stable and non-toxic but they must also have physical properties that mimic the bodily tissue they are replacing, as in reconstructive surgery, or mimic the bodily tissue in the proximity of the endoprosthesis, as in cosmetic surgery.

Materials such as collagen are resorbed into the body over short periods of time. Silicone and Soya have encountered serious safety issues. There is currently a need for a safe, stable, biocompatible material that possesses the physical properties to mimic soft tissue.

The present inventors have surprisingly found that a polyacrylamide hydrogel comprising less than 3.5% polyacrylamide, based on the total weight of the hydrogel, is an effective endoprosthesis with advantageous physical properties. Contrary to US 5,798,096, the polyacrylamide gel is stable. The hydrogel, as prepared by a process according to the present invention, is cross-linked with methylene bis-acrylamide to a degree such that the endoprosthesis prepared from said hydrogel possesses advantageous physical characteristics. The hydrogel according to the present invention is a new chemical entity as indicated by its novel and advantageous physical characteristics. These secondary characteristics are indicative that the degree of cross-linking in the hydrogel of the present invention differs greatly from polyacrylamide hydrogels prepared by disclosed processes. This degree of cross-linking is a critical contributor to its physical properties.

The present investigators have provided a bio-stable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. The bio-stable hydrogel typically has a molecular weight between 0.01 x 10⁶ and 20 x 10⁶. The polymer is resistant to biological, degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

Alternatively defined, one aspect of the invention relates to hydrogel comprising i) less than 3.5 % polyacrylamide by weight, based on the total weight of the hydrogel, cross-linked with methylene bis-acrylamide and ii) at least 95% pyrogen-free water or saline solution.

A further aspect of the invention relates to a hydrogel for use as an injectable or implantable endoprosthesis said hydrogel comprising i) less than 3.5% polyacrylamide by weight, based on the total weight of the hydrogel, cross-linked with methylene bis-acrylamide and ii) at least 95% pyrogen-free water or saline solution.

In all embodiments wherein the hydrogel comprises less than 3.5% polyacrylamide by weight, based on the total weight of the hydrogel, the hydrogel typically further comprises at least 95% pyrogen-free water or saline solution.

The hydrogel comprises less than 3.5% polyacrylamide preferably comprising at least 0.5%, such as at least 1 %, preferably at least 1.5% polyacrylamide, such as at least 1.6% polyacrylamide by weight, based on the total weight of the hydrogel.

The hydrogel comprising less than 3.5% polyacrylamide are chemically stable and bio-stable but may be very fluid such that they may be characterised in that it has a complex viscosity not less than 2 Pas, such as not less than 3, 4 or 5 Pas. In a suitable embodiment, the hydrogel comprising less than 3.5% polyacrylamide is characterised in that it has complex viscosity from 2 to 90, such as 5 to 80 Pas, preferably from 6 to 76, such as from 6 to 60, 6 to 40, 6 to 20, such as 6 to 15 Pas.

The hydrogel comprising less than 3.5% polyacrylamide has elastic properties in that the hydrogel may be characterised in that it has elasticity module of not less than 10 Pa, such as not less than 20, 25, 30, 31, 32, 33, 34 or 35 Pa, such as not less than 38 Pa. Typically, the hydrogel has an elasticity module from 10 to 700 Pa, such as 35 to 480 Pa.

These rheological features are in due in part to the degree of cross-linking and to the degree of swelling of the hydrogel. The hydrogel comprising less than 3.5% polyacrylamide may be characterised in that the cross-linked polyacrylamide is to such as degree so as to have an efficient cross-linking density of 0.2 to 0.5%, preferably 0.25 to 0.4%.

The cross-linking density is in turn due in part to the molar ratio between the acrylamide and methylene-bis-acrylamide. Typically said ratio is in the range 175:1 to 800:1, such as from 225:1 to 600:1, preferably from 250:1 to 550:1, most preferably from 250:1 to 500:1. The absolute and relative amount of the redox agent (TEMED) and the initiator also influence the degree of cross-linking. As can be seen from Tables 1, 2, 3, 4, the present investigators have adjusted these parameters to influence the rheological properties of the hydrogel.

The biocompatible hydrogels of the invention comprising less than 3.5% polyacrylamide may be suitably characterised, at least in part, by one or more of the following features: i) a cross-linking density of 0.2% to 0.5 %; ii) an elasticity modulus (G') of 10 to 700 Pa; iii) a complex viscosity of 2 to 90 Pa s; iv) a dry matter content of less than 3.5% such as less than 3.4, such as less than 3.3, such as less than 3.2, such as less than 3.1, such as less than 3.0, such as less than 2.9, such as less than 2.8, such as less than 2.7, such as less than 2.6 Pa s; v) a refractive index between 1.33 and 1.34.

As stated, in all aspects of the invention wherein the hydrogel comprises less than 3.5% polyacrylamide by weight, based on the total weight of the hydrogel, the hydrogel further comprising at least 95% pyrogen-free water or saline solution. In all embodiments wherein the hydrogel further comprises saline solution, the hydrogel preferably comprises less than 3 % polyacrylamide by weight, based on the total weight of the hydrogel.

The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

The hydrogel comprises pyrogen-free water or saline solution. The combining of the reagents and the casting of the gel may therefore be done in pyrogen-free water or saline solution. The use of saline solution will clearly increase the total solid weight content of the hydrogel but does not significantly influence the polyacrylamide content during the polymerisation reaction.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is an 0,8- 1 % aqueous sodium chloride solution, such as a 0.8, 0.9 or 1 % aqueous sodium chloride solution.

As stated, pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm. In the method of the invention, the washing step comprises swelling the product for 50 to 250 hours, more typically for 70 to 200 hours.

The hydrogel comprising less than 3.5% polyacrylamide was surprisingly found to be stable at very low solid weight content, contrary to the teachings of US 5,798,096. Polyacrylamide hydrogels with a solid-weight content of 0.5% have been prepared by the present investigators. Preferred embodiments of the hydrogel of the invention comprise at least 0.5%, such as at least 1 %, preferably at least 1.5% polyacrylamide, such as at least 1.6% polyacrylamide by weight, based on the total weight of the hydrogel.

The hydrogel is suitably a composite of cross-linked polyacrylamide chains and pyrogen-free water. Water contained in the hydrogel as part of a composite is loosely bound with the polymer chains. When present in a body, some of the water molecules move into the tissue by osmosis resulting in a smoothing out of the affected skin surface. In the embodiment wherein the hydrogel comprises saline solution, the iso-osmolarity of the saline solution with interstitial fluid minimises immune responses.

As mentioned, the physical properties of the hydrogel are influenced in part by the degree of cross-linking. The degree of cross-linking may be controlled in part by the molar ratio of cross-linking agent, methylene bis-acrylamide, to acrylamide.

Therefore, another object of the invention is a method for the preparation of a hydrogel comprising the steps of combining acrylamide and methylene bis-acrylamide, radical initiation, and washing with pyrogen-free water or saline solution so as to give less than 3.5% by weight polyacrylamide, based on the total weight of the polyacrylamide. The method is preferably such that the hydrogel comprises at least 0.5%, such as at least 1 %, preferably at least 1.5% polyacrylamide, such as at least 1.6% polyacrylamide by weight, based on the total weight of the hydrogel.

In a especially preferred embodiment of the invention, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution.

The combining of acrylamide and methylene bis-acrylamide in preferably done in a molar ratio between acrylamide and methylene bis-acrylamide is from 175:1 to 800:1, such as from 225:1 to 600:1, preferably from 250:1 to 550:1, most preferably from 250:1 to 500:1.

An illustrative preparation of the hydrogel according to the present invention is described in Example 1. The hydrogel having the desired physical properties has been obtained by combining acrylamide and methylene bis-acrylamide in a ratio of 250:1, such as 252:1, 254:1, 256:1, 258:1 and 260:1, as well as by combining acrylamide and methylene bis-acrylamide in a ratio of 500:1, such as 498:1, 496:1 494:1 492:1, or 490:1. The hydrogel according to the present invention preferably has a complex viscosity from 2 to 90, such as 5 to 80 Pas, typically from about 6 to 76, such as from 6 to 60, 6 to 40, 6 to 20, such as 6 to 15 Pas. In a suitable embodiment, the washing step comprises swelling the product of the radical initiation step until the complex viscosity is from 6 to 100 Pas.

In a suitable embodiment of the invention, the hydrogel has a degree of cross-linking such that it has complex viscosity of not less than 2 Pas, such as not less than 3, 4 or 5 Pas, such as not less than 5.5 Pas, such as not less than 6 Pas, preferably not less than 6.2 Pas.

The elasticity module is an alternative physical characteristic of the hydrogel indicative, in part, of the degree of cross-linking of the hydrogel according to the present invention. Typically, the degree of cross-linking is such that the hydrogel has an elasticity module of not less than 10 Pa, such as not less than 25, 30, 31, 32, 33, 34 or 35 Pa, such as not less than 38 Pa. The gel may be characterised in that it has elasticity module from 10 to 700 Pa, such as 35 to 480 Pa.

As stated, in a most preferred embodiment, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution. The radical initiation step yields a hydrogel still comprising toxic reagents and not yet possessing the beneficial physical properties of the hydrogel of the present invention. The washing step comprises swelling the hydrogel produced from the radical initiation step until the complex viscosity is from 2 to 90 Pas.

Alternatively measured, the washing step may comprise swelling the product of the radical initiation step until the elasticity module is elasticity module from 10 to 700 Pa, such as from 35 to 480 Pa.

By nature, a low degree of cross-linking typically results in a higher swelling rate consequently lowering the dry matter content (percentage acrylamide), as well as lowering the elasticity module and viscosity. Thus, in addition to the degree of cross-linking, the time the hydrogel is exposed to the washing step influences to a degree the physical properties of the gel.

Typically, the washing step comprises swelling the product for 80 to 100 hours, such as 90-95 hours. This usually results in an increase in weight of the hydrogel of 75 to 150%, usually around 100% increase.

Furthermore, the amounts of the free-radical initiator in the radical initiation step and the amount of co-initiator influences the chain length and thus the physical properties of the hydrogel. In a typical preparation of the hydrogel, N-,N-,N-,N-tetramethyl ethylene diamine (TMED) is used as the co-initiator and ammoniumpersulfate (APS) is used as the free-radical initiator (redox-system). Adequate amounts of initiator and co-initiator are required to obtain the hydrogel according to the invention. As an illustration, an insufficient amount of these reagents will result in shorter chain lengths and consequently influence the degree of cross-linking and hence the physical properties of the hydrogel. Other reaction conditions, such as temperature also influence chain length.

As stated, the degree of cross-linking influences the physical properties of the hydrogel. The degree of cross-linking of the hydrogel of the present invention may be indirectly measured, as described above, by the elasticity module and/or by the complex viscosity. An alternative measure of the degree of cross-linking of the hydrogel is its efficient cross-linking density. The hydrogel of the present invention preferably comprises 1.6 to 3.5% (wt/wt) polyacrylamide cross-linked with methylene bis-acrylamide and ii) pyrogen-free water orsaline solution, preferably such that the degree of cross-linking as measured by its efficient cross-linking density is such that the efficient cross-linking density is 0.2 to 0.5%, preferably 0.25 to 0.4%.

In a suitable embodiment of the present invention, the hydrogel may comprise 1.6 to 3.25% (wt/wt) polyacrylamide, such as 1.8 to 3.1, 2.0 to 3.0, 2.0 to 2.9, preferably 2.0 to 2.8 (wt/wt) polyacrylamide.

### Medical Use of Polyacrylamide Hydrogels

The hydrogels of the invention are intended for use as endoprosthetic devices. The endoprosthetic devices of the invention and methods of treatment described herein may use the hydrogel in any of the embodiments described supra.

The endoprosthetic device may be administered to the body of an individual by means of injection, such as through a syringe or catheter or by means of surgical implantation. In the embodiment wherein the hydrogel is for use as an implantable endoprosthesis, the hydrogel may optionally serve as filler material in an envelope, the whole of which is implanted into the body. Thus the hydrogel or the endoprosthesis may comprise a silicone-based envelope housing the hydrogel.

As stated, an object of the invention is a method of treatment of a cosmetic or functional defect with an injectable biocompatible endoprosthesis comprising:
a) preparation of a 1.6 to 3.5% by weight polyacrylamide hydrogel, said polyacrylamide cross-linked using methylene bis-acrylamide,
b) injection a sufficient amount of said hydrogel into a region of the body affected by a cosmetic orfunctional defect. The hydrogel injected into an affected area is obtainable by combining acrylamide and methylene bis-acryalmide in amounts so as to give 1.6 to 3.5% by weight polyacrylamide and in a molar ratio of 150:1 to 1000:1, radical initiation, and washing with pyrogen-free water or saline solution. The ratio of acrylamide to methylene bis-acrylamide is preferably such that the complex viscosity is from 2 to 90, such as 5 to 80 Pas, preferably from 6 to 76 Pas or such that the elasticity module is from 10 to 700 Pa, such as 35 to 480 Pa. The washing step may be done to the extent such that the complex viscosity is from 6 to 80 Pas or such that the elasticity module is from 10 to 700 Pa, such as 35 to 480 Pa.

The endoprosthetic device may comprise any of embodiments of the hydrogels discussed herein and may be either implantable or injectable Thus an important aspect of the present invention is the use of a hydrogel comprising i) less than 3.5% by weight polyacrylamide cross-linked with methylene bis-acrylamide and ii) at least 95% pyrogen-free water or saline solution for the preparation of an endoprosthesis for cosmetic surgery, reconstructive surgery and therapy. In particularly interesting embodiments of invention, the endoprosthesis is injectable.

A further object of the invention is a hydrogel as described *supra* for use in the preparation of an endoprosthesis. Specifically, an object of the invention is the use of a hydrogel comprising i) 1.6 to 3.5% by weight polyacrylamide cross-linked using methylene bis-acrylamide and ii) pyrogen-free water or saline solution for the preparation of an endoprosthesis for cosmetic surgery, reconstructive surgery and therapy. The endoprosthesis according to the present invention may be implantable or injectable.

A hydrogel as described supra may be used for the preparation of an endoprosthesis. Thus, a further object of the present invention is an injectable endoprosthesis comprising i) 1.6 to 3.5% by weight polyacrylamide cross-linked with methylene bis-acrylamide and ii) pyrogen-free water or saline solution. An injectable endoprosthesis according to the present invention preferably has a viscosity complex from 2 to 90, such as 5 to 80 Pas, preferably from 6 to 76, such as from 6 to 60, 6 to 40, 6 to 20, such as 6 to 15 Pas.

Wherein the endoprosthesis is injected, the hydrogel is generally somewhat fluid in nature.

Many disorders are related to a loss of effective activity of the tissue at a functional interface between two organs. For instance, urinary incontinence is related to insufficient sphincter between the urinary bladder and the urethra. By injecting or implanting an endoprosthesis prepared from the hydrogel according the present invention into the proximal submucosa of the urethra, thereby narrowing the urethra, the disorder may be significantly controlled. Similarly, reflux oesophagus is related to insufficient resistance between the oesophagus and stomach. By injecting or implanting an endoprosthesis prepared from the hydrogel according the present invention along the sphincter between the oesophagus and stomach, the contact between the contents of the stomach and the oesophagus may be reduced. Thus, in suitable embodiments, the hydrogel is used for the preparation of an endoprosthesis treat urinary incontinence or reflux oesophagitis. The endoprosthesis may, in general, be used to treat disorders related to insufficient resistance at a functional interface between two organs or between segments of one organ.

The solid-weight content of weight percentage acrylamide, as measured after the washing step, as well as the degree of cross-linking is adapted according to the use of the endoprosthesis prepared from the hydrogel. In preferred embodiments, the endoprosthesis is preferably prepared from a hydrogel comprising 1.6 to 3.25% (wt/wt) polyacrylamide, such as 1.8 to 3.1, 2.0 to 3.0, 2.0 to 2.9, preferably 2.0 to 2.8 (wt/wt) polyacrylamide. Alternatively defined, the degree of cross-linking of the hydrogel for use as an endoprosthesis may preferably be such that the complex viscosity of the hydrogel is from 2.0 to 15 Pas such as from 5.5 to 15 Pas, such as from 6 to 12 Pas. Alternatively measured, for facial corrections the degree of cross-linking of the hydrogel are preferably such that the elasticity module is from 10 to 100 Pa, such as 35 to 75, particularly 35 to 60 Pa, such as 35 to 50 Pa.

The endoprosthesis may be to correct a defect which is the result of trauma such as tumours or physical injury as well as congenital defects.

In a suitable embodiment of the present invention, the hydrogel as described *supra* is used as a filling material to fill or partially fill a silicone-based reservoir or shell for the preparation of an implantable endoprosthesis. Thus, a further object of the present invention is an implantable endoprosthesis comprising i) a hydrogel comprising 1.6 to 3.5% (wt/wt) polyacrylamide cross-linked with methylene bis-acrylamide and pyrogen-free water or saline solution ii) a silicone-based shell adapted for containing said hydrogel. The hydrogel used to fill or partially fill the silicone-based shell may be a hydrogel obtainable by combining acrylamide and methylene bis-acryalmide in amounts so as to give 1.6 to 3.5% by weight polyacrylamide and in a molar ratio of 150:1 to 1000:1, radical initiation, and washing with pyrogen-free water or saline solution. In embodiments wherein the endoprosthesis is implantable, the preparation of said endoprosthesis from a hydrogel optionally further comprises the step of inserting said hydrogel into a silicone-based shell.

After the hydrogel has been placed into the silicone-based shell, it can preferably be sealed by chemical, mechanical or thermal means or by laser or light. Typically, the outer surface of the shell is chemically or physically modified such that it is biocompatible and have a friction coefficient so as to minimise movement or slippage from the position it was placed.

In an alternative embodiment of the invention, the endoprosthesis may comprise a medicament for use in therapy.

### Polyacrylamide Hydrogel For The Treatment Of Incontinence And Vesicouretal Reflux

The loss of voluntary control of sphincter muscles underlies urinary and anal incontinence. Urinary incontinence, the inability to voluntarily retain one's urine in one's bladder, is common among the elderly. Contraction of the detrusor muscle of the bladder may be voluntary, brought about stress, reflex or urge. Contraction of the detrusor muscle brought about by a stress results in involuntary urination unless the bladder sphincter muscle is voluntarily contracted. In the event that one has lost control of the bladder sphincter muscle, urination may be brought about by slight stress such as slight abdominal contractions during daily activities, sneezing, coughing, laughing, gas retention, surprises and countless other stimuli which may lead to contraction of the detrusor muscle. A similar principle underlies anal incontinence wherein one has diminished control of the anal sphincter muscle. Urinary and anal incontinence may be brought upon by ageing, trauma (such as in paraplegics), or congenitally related.

Vesicouretal reflux is the result of decreased ureteral resistance wherein urine from the bladder refluxes back into the kidney. This can result in the transport of bacteria form the bladder back up through the ureter, clayceal dilation, the renal pyramids and the kidneys and may lead to infections and recurrent pyelonephritis as well as cause physiological injury to the renal parenchyma. This may lead to renal failure.

Urinary incontinence, anal incontinence and vesicouretal reflux may be treated by increasing the resistance of passage through the urethra, the colon or rectum (or *canalis analis*), and the ureter, respectively, known as bulking processes

Attempts to treat urinary incontinence have involved hydraulic apparatuses, such as in WO 01/50833 and US 4,969,474 and other controllable apparatuses such as prosthetic sphincters with inflatable cuffs as in US 4,571,749 and WO 01/47433. Weight loss, exercise, medication and surgical operation usually involving the elevation of the bladder neck or constructing a increasing resistance through the urethra using the surrounding tissue or using a prosthetic material. Materials such as collagen, PTFE, silicone, and Teflon have been used to this end (references to be added).

Anal incontinence has also been addressed via controllable and operated devices such as in WO 01/47431. Vesicouretal reflux has been dealt with surgically (to extend the ureter, usually in the infant) and with antibiotics.

A principle object of this aspect of the invention is to provide a polyacrylamide hydrogel to increase the resistance in the relevant conduits in the treatment of incontinence and vesicouretal reflux.

US 6,129,761 discloses the use of injectable hydrogel and cellular compositions towards this end. The compositions are cell suspensions comprising cells mixed with a biocompatible and biodegradable polymer. The polymer provides a medium and template for cell growth and cellular engraftmentto the surrounding tissue. Cell growth coincides with polymer degradation to result in the desired tissue growth. The polymeric materials disclosed by US 6,129,761 consist of alginates such as modified alginates, bacterial polysaccharides such as gellan gum, plant polysaccharides such as carrageenans, hyaluronic acids, polyethylene oxide-polypropylene glycol block copolymers, proteins such as fibrin, collagen, and gelatin, mixtures of polyethylene oxide and polyacrylic acid, cross-linked chitosan, photochemically cross-linked ethylenically unsaturated groups, macromers such as PEG-oligolactyl-acrylates, polyethylenimine, polylysine, poly(vinylamine), and poly(allylamine). US 6,129,761 does not disclose the use of stable polyacrylamide hydrogels but rather degradable poly(vinylamine). Given the requirement of the polymer of the invention of US 6,129,761 to degenerate according to the method of the invention, biostable polymers, such as the biostable polyacrylamide of the present invention is not suitable for the method of US 6,129,761.

RU2148957 relates to a method for treating cases of vesicoureteral reflux using polyacrylamide hydrogel.

The substances for injection used thus far used are synthetic substances (teflon, silicone, coal particles) and natural substances (connective tissue extract, fat). The long-term effect has been relatively poor with relapses of incontinence among approximately half the treatments after one year of observation. Thus, there is a need to find a substance which is biocompatible with tissue but at the same time is not absorbed or excreted by the body (biostable). Moreover, the substance must have adequate rheological properties to act effectively.

An object of this aspect of the invention is to provide a biocompatible and bio-stable polyacrylamide hydrogel for increasing the resistance of conduits for the treatment of incontinence and vesicouretal reflux.

The term "bio-stable" is intended to mean that the substance does not degrade to any substantial degree in interstitial fluid or body tissue.

A first aspect of this aspect of the invention relates to the bio-stable hydrogel obtainable by combining acrylamide and methylene bis-acrylamide in amounts so as to give 0.5 to 25% by weight polyacrylamide, based on the total weight of the hydrogel; radical initiation; and washing with pyrogen-free water or saline solution, for use in the in the treatment and prevention of incontinence and vesicouretal reflux. The bio-stable hydrogel typically has a molecular weight between 0.01 x 10⁶ and 20 x 10⁶. The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble.

The present investigators have found that the bio-stable gel is effective as a bulking agent and suitable for the treatment of incontinence and vesicouretal reflux given the gel is biocompatible, non-biodegradable and does not migrate from the site of application in the conduit.

The bio-stable hydrogel of this aspect of the invention is typically obtained by combining acrylamide and methylene bis-acrylamide in a molar ratio of 150:1 to 1000:1. In a preferred embodiment, the hydrogel comprises less than 15% by weight polyacrylamide, based on the total weight of the hydrogel, preferably less 10%, more preferably less than 7.5%, even more preferably less than 5%, most preferably less than 3.5% by weight polyacrylamide, based on the total weight of the hydrogel.

Given the hydrogel of this aspect of the invention is directed for use as a permanent endoprosthesis, it must be stable. Furthermore, given the hydrogel of the invention is directed for use as an endoprosthesis for selected parts of the human anatomy, the hydrogel comprises at least 0.5 % by weight polyacrylamide, based on the total weight of the hydrogel, preferably at least 1.0 % by weight polyacrylamide, more preferable at least 1.5 % by weight polyacrylamide, such as at least 1.6 % by weight polyacrylamide, based on the total weight of the hydrogel. Typically, the hydrogel of the present invention may have a solid weight content of 1.5. 1.6, 1.7 1.8, 1.9, 2.0, 2.1, 2.2, 2.3, 2.4, 2.5. 2.6, 2.7. 2.8. 2.9, 3.0, 3.1, 3.2, 3.3, 3.4. or 3.5 % polyacrylamide, based on the total weight of the hydrogel.

For the treatment of vesicouretal reflux, the hydrogel comprises less than 3.5% by weight polyacrylamide, based on the total weight of the hydrogel and about 96.5% pyrogen-free water of saline solution, such as 97.5% pyrogen-free water of saline solution.

The viscosity of the bio-stable hydrogel is typically such that it may be injected. In a suitable embodiment, the hydrogel has a complex viscosity of 2 to 50 Pas, such as 2 to 40 Pas, preferably 2 to 30 Pas, more preferably 2 to 20 Pas.

The device may have a viscosity such that it may be injected. In an embodiment wherein the hydrogel is injected, the hydrogel has a complex viscosity of 2 to 30 Pa s, such as about 2 to 20 Pa s, preferably about 3 to 18 Pa s, most preferably about 3 to 15 Pa s, such as 3, 4, 5. 6. 7, 8. 9, 10, 11, 12, 13, 14, and 15 Pa s.

The device also has elastic properties due to, at lest in part of the high water binding capacity of the hydrogel of water. This is of great relevance in terms, at least, of durability and ability to provide resistance through the conduit. In a preferred embodiment, the hydrogel of the invention has an elasticity modulus of 1 to 200 Pa, such as 2 to 175 Pa, typically 5 to 150 Pa, such as 10 to 100 Pa.

The elasticity modulus of the prosthetic device and the complex viscosity are typically related by a factor of 5.8 to 6.4. The present invention thus provides for a hydrogel with the advantageous combined features of a viscosity suitable for being injectable and of an elasticity to provide for increase resistance. In a combination of preferred embodiments, the hydrogel has a complex viscosity less than 25 Pa s and an elasticity modulus less than 200 Pa, preferably having a complex viscosity less than 15 Pa s and an elasticity less than 100.

Example 1, Tables 1, 2. 3 and 4 illustrate appropriate conditions for preparing the illustrative examples of hydrogels. As can be seen, within the preferred combined embodiment of a viscosity of less than 25 Pa s and an elasticity modulus of less than 200 Pa, the hydrogel may have an array of dry-weight content percentages.

Furthermore, still within the preferred combined embodiment of a viscosity of less than 25 Pa s and an elasticity less than 200 Pa, such as having a complex viscosity less than 15 Pa s and an elasticity less than 100, the hydrogel is obtainable by combining acrylamide and methylene-bis-acrylamide in a molar ratio of 275 to 1000, typically 300 to 800, preferably in a ratio of 300 to 500.

The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85%, more preferably at least 90%, even more preferably at least 95% by weight pyrogen-free water or saline solution.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 -1 % glucose solution, a potassium chloride solution, and a calcium chloride solution. In a preferred embodiment, the saline solution is an 0.8-1 % aqueous sodium chloride solution, such as a 0.8, 0.9 or 1 % aqueous sodium chloride solution.

In a particularly suitable embodiment of this aspect of the invention, the hydrogel comprises about 2.5 % by weight polyacrylamide, based on the total weight of the hydrogel and about 97.5 % pyrogen-free water.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm.

The washing process may suitably be performed for 15 to 250 hours, such as for 20 to 225 hours. From Table 2 and 3, it can be seen that the washing process is typically performed for 50 to 100 hours, more typically for 70 to 100 hours.

In a further embodiment of this aspect of the invention, the device prosthetic device comprises cells, such as stem cells. Polyacrylamide provides an excellent template and matrix for cell growth. The use of cells with the hydrogel of the invention for the preparation of the device would allow for cellular engraftment to the surrounding tissue in the ureter, urethra or *analis canalis.* A device comprising the hydrogel of the invention and the appropriate cells allows for greater resistance and greater efficiency in providing resistance.

In a preferred embodiment of this aspect of the invention, the hydrogel of the invention is for use in the treatment of urinary and anal incontinence, more preferably urinary incontinence.

Urinary incontinence may be stress or reflex urinary incontinence or urge urinary incontinence. Typically, the hydrogel of the invention is suitable for the treatment of stress or reflex urinary incontinence.

In a further object of this aspect of the invention, the present hydrogel is used in the preparation of an endoprosthesis. Thus, a further object of the invention is the use of a hydrogel, as described *supra,* comprising 0.5 to 25% by weight polyacrylamide, based on the total weight of the hydrogel, for the preparation of an endoprosthesis for the treatment and prevention of incontinence and vesicouretal reflux.

The endoprosthesis is suitably formulated as an injectable suspension. The suspension comprises a homogenised formulation of the hydrogel. Typically, a syringe is filled with the suspension.

A further object of this aspect of the invention relates to a method of treating or preventing incontinence or vesicouretal reflux comprising administering a hydrogel to a mammal said hydrogel comprising 0.5 to 25% by weight polyacrylamide, based on the total weight of the hydrogel. The hydrogel, in any of the above-described embodiments, is suitable for the method of the invention.

Upon administration of the hydrogel, a thin layer of connective tissue surrounds the endoprosthesis, enabling the endoprosthesis to become a stable part of the connective tissue. Due to the stability of the hydrogel and the thin layer of connective tissue, the endoprosthesis may be removed from the patient. This advantage is at least in part due to the stability of the hydrogel which in turn is at least in part due to the washing process.

Several factors affect the rheological properties of the hydrogel, such as the relative amount of monomer used, the relative amount of initiator, the temperature and other parameters of the polymerisation process, and the washing process. Thus, the polymerisation process may provide a hydrogel with an array of viscosities. The invention is directed to an endoprosthesis typically for the urethra, the rectum or colon (or *canalis analis*), or the ureter and may thus be tailored to the requirements of the conduit.

An important object of the invention is to provide a prosthetic device for increasing the resistance of conduits selected from the group consisting of the urethra; the rectum or colon (or *canalis analis*); and the ureter for the treatment of urinary incontinence, anal incontinence, and vesicouretal reflux, respectively; wherein said device is injectable and comprising the hydrogel as described herein.

The method of this aspect of the invention preferably includes the administering of the hydrogel by means of injecting the hydrogel into the appropriate conduit. In the treatment of urinary incontinence, the hydrogel is typically injected into the urethra, specifically under the submucosal membrane of the urethra. Injection is via the external surface of the urethra and toward the submucosal membrane.

The present investigators have found that typically 2 to 5 mL of the hydrogel are suitable to provide adequate resistance in the urethra by bulking the urethra. Typically, 3 mL of hydrogel is injected and preferably the 2-5 mL are distributed by depositing the gel at more than one cross-sectional position along a single longitudinal position of the urethra. In a particularly suitable embodiment, 3 or more depots are made along a single longitudinal position of the urethra. The present investigators have found that depots 0.5 cm distally from the neck of the bladder are particularly suitable.

The present investigators have found that submucosal injections at positions 10, 2, and 6 o'clock of the cross-sectional axis of the urethra to be particularly suitable for the treatment of urinary incontinence.

The depots are typically made by means of a syringe or by use of a cytoscope or catheter. Suitably a 21 to 27G needle is employed for the injection.

For the treatment of anal incontinence, the hydrogel is typically injected into the colon or rectum (*canalis analis*) specifically under the submucosal membrane of the colon or rectum. Injections of 2 to 6 ml are suitable. The hydrogel is preferably distributed at more than one cross-sectional position along a single longitudinal position of the colon or rectum. In a particularly suitable embodiment, 3 or more depots are made along a single longitudinal position of the colon or rectum, preferably at positions 10, 2, and 6 o'clock of the cross-sectional axis of the colon or rectum.

For the treatment of vesicouretal reflux, submucosal injections into the ureter of the patient is required. Injections of 2 to 5 ml are suitable. The hydrogel is preferably distributed at more than one cross-sectional position along a single longitudinal position of the ureter In a particularly suitable embodiment, 3 or more depots are made along a single longitudinal position of the ureter, preferably at positions 10, 2, and 6 o'clock of the cross-sectional axis of the ureter.

In an alternative embodiment of this aspect of the invention, the method comprises the use of a prosthetic device comprising cells, such as stem cells. Polyacrylamide provides an excellent template and matrix for cell growth. The use of cells in combination with the hydrogel of the invention for the preparation of the device would allow for cellular engraftmentto the surrounding tissue in the ureter, urethra or *analis canalis.* A method comprising the hydrogel of the invention and the appropriate cells allows for greater resistance and greater efficiency in providing resistance.

### EXAMPLES

### Example 1

### Preparation of Hydrogel

The gel is manufactured by a polymerisation of the monomers of acrylamide and N,N'-methylene-bis-acrylamide. The finished product may have different viscosities.

The hydrogel typically contains approximately 95% water. The concentration of the monomers acrylamide and N,N'-methylene-bis-acrylamide has been shown to be less than 10 ppm and is adequate for the desired stability of the final product, often less than 5 ppm.

The finished product must conform with respect to pH, absence of heavy metals, refractive index, stability, absence of pyrogens, and must be sterile, practically inert, and be substantially free of monomers.

### Preparation 1.1

The synthetic preparation suitably involves the following operations:
1. Two mixtures, A1 and A2, are prepared. A1 comprises water, acrylamide, N,N'-methylene-bis-acrylamide, N,N, N',N'-tetramethylene-ethylene-diamine (TEMED). A2 comprises water and ammonium persulphate;
2. The two mixtures are combined in the following ratio: 1990 mL of A1 and 10 mL of A2 and kept at 45 °C and degassed with nitrogen for 20 seconds;
3. The reaction mixture is cast into several 100 mL beakers;
4. Polymerisation is allowed to occur for 0.5 to 1.5 hours;
5. The gel is demolded;
6. Residual monomers are extracted and with equilibration in W FI water for 92 hours, changing the water several times, typically 8 times during the 92 hours;
7. The purified gels are homogenised by grinding with an vertically oscillating grid;
8.The syringe is filled with the homogenised gel material;
9. Autoclavation of the syringe

A typical method for preparing the hydrogel may be summarised as:

### Preparation 1.2

*Process summary.* The gel is prepared by mixing an aqueous monomer solution of acrylamide (AM) and N, N'-methylene-bis-acrylamide (BISAM) as cross-linker with N,N,N',N'-tetramethylene ethylene diamine (TMED) as co-initiator and ammoniumpersulfate (APS) as free-radical initiator (redox-system). By degassing a bulk solution with nitrogen polymerisation starts. After final polymerisation the gel transferred into a washing tank with net trays onto which the gel is placed. During water washing the gel swells and monomer residues are extracted. The swollen gel is fed and evacuated in a filling unit having the gel delivered in a syringe, which is autoclaved.

Two alternate formulations have been prepared, a lower- and a higher- end viscosity formulation. Both formulations have a solid weight content of less than 3.5% and a complex viscosity in the range of 2 to 50 Pa s, typically between 3 and 20 Pa s.

**Table 1**

| Chemical constituent | lower end viscosity | higher end viscosity |
|---|---|---|
| acrylamide | 502 g | 547 g |
| N,N'-methylene-bis-acrylamide | 2,2 g | 4,6 g |
| TMED | 3,0 g | 2,6 g |
| APS | 5,4 g | 5,0 g |
| Non-pyrogenic water | Add 10 litre | Add 10 litre |

The above are typical preparations of the hydrogel and may be adjusted within certain ranges.

### Preparation 1.3

### Polyacrylamide formulations from inline cross-linking process

A particularly interesting method of preparing the hydrogels of the invention involves an inline cross-linking process. Two individual and eventually degassed flows, one being a pre-mix of acrylic amide, bis-methylene acryl amide (the cross-linker) and TEMED, the other being the AMPS initiator solution, are pumped into a static mixer for mixing, chemical initiation and subsequent extrusion downstream into a pipe reactor made of Teflon or steel in which the polymerisation occurs. Washing of the gel is simplified due to high surface area of gel from reactor.

By selecting monomer, cross-linker and initiator concentrations and their relative molar ratios, and by regulating the two flow rates and the polymerisation temperatures, it is possible to produce gels that are varying in degree of crosslinking and in solids content.

### Preparation 1.4

The reagents were combined in ratios described in Tables 2, 3 and 4, and washed as described in the Tables (with pyrogen-free water unless indicated otherwise) to give low, medium, and high viscosity formulations. Hydrogels with solid weight contents between 0.5 and 25% polyacrylamide were prepared.

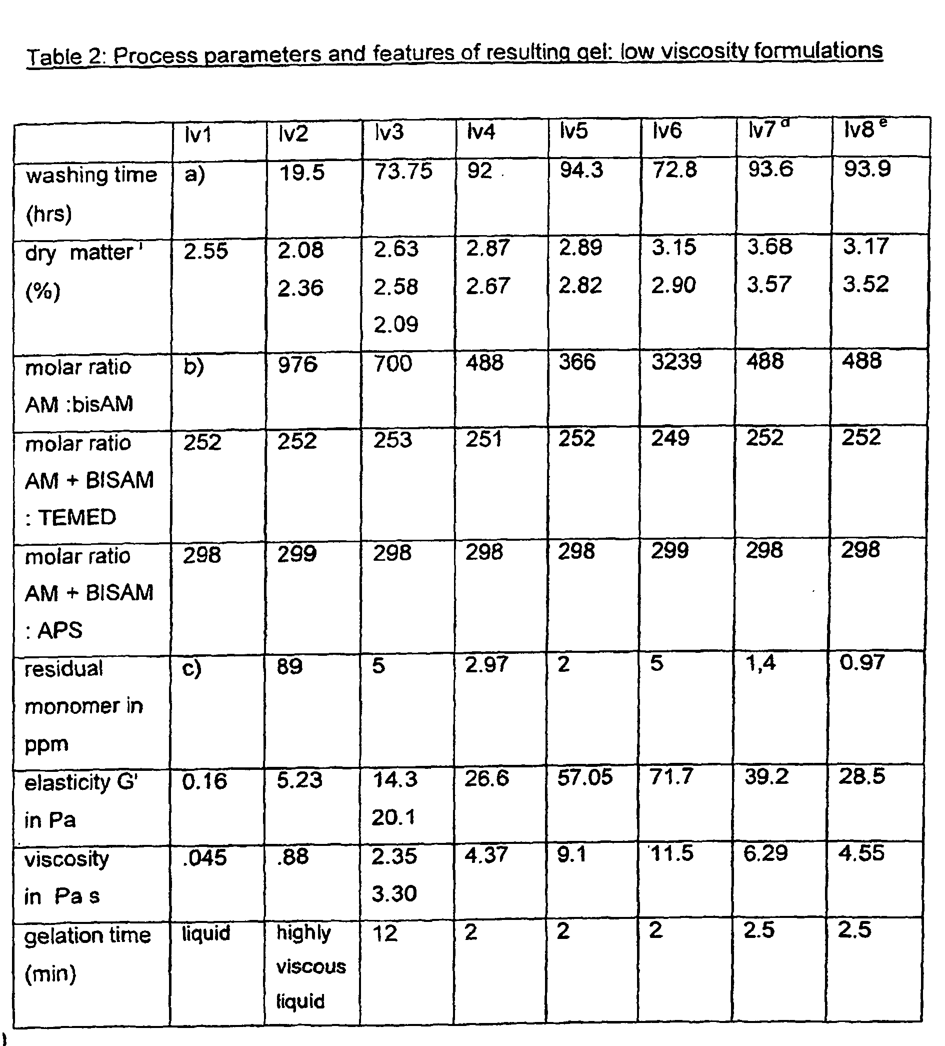

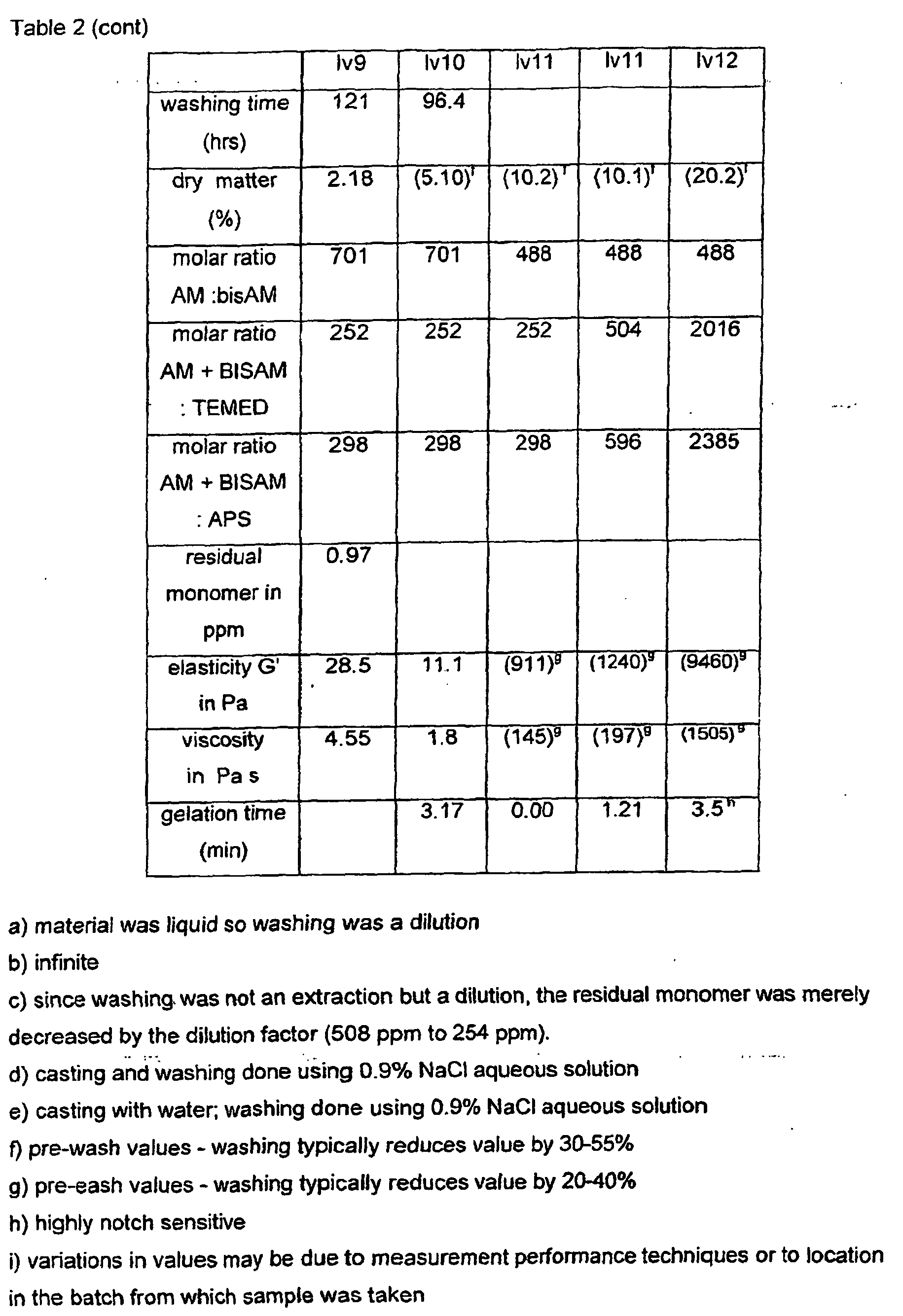

**Table 3:**

| Process parameters and features of resulting gel: medium viscosity formulations | | | | | |
|---|---|---|---|---|---|
| | mv1 | mv2 | mv3 | mv4 | mv5 |
| washing time (hrs) | 97 | 211.5 | 96 | 94.8 | 90.3 |
| dry matter (%) | 3.14 | 2.49 | 3.25 | 3.29 | 3.22 |
| molar ratio AM :bisAM | 310 | 310 | 290 | 289 | 289 |
| molar ratio AM + BISAM :TEMED | 252 | 252 | 252 | 251 | 252 |
| molar ratio AM + BISAM : APS | 299 | 299 | 299 | 299 | 299 |
| residual monomer in ppm | 1.6 | | 1.5 | | |
| elasticity G' in Pa | 108.5 | | 129 | 133.5 | |
| viscosity in Pa s | 17.4 | | 20.6 | 21.30 | |
| gelation time (min) | 2.5 | 2.5 | 2.18 | | |

**Table 4:**

| Process parameters and features of resulting gel: high viscosity formulations | | | | | |
|---|---|---|---|---|---|
| | hv1 | hv2 | hv3 | hv4 | hv5 |
| washing time (hrs) | 119.5 | 516 | 122 | 95.5 | 116.7 |
| dry matter (%) | 3.47 | 2.5 | 3.56 | 3.83 | 3.42 |
| molar ratio AM :bisAM | 260 | 260 | 260 | 260 | 260 |
| molar ratio AM + BISAM : TEMED | 315 | 315 | 604 | 313 | 314 |
| molar ratio AM + BISAM : APS | 376 | 376 | 755 | 375 | 376 |
| residual monomer in ppm | 0.2 | | | | |
| elasticity G' in Pa | 343 | 274 | | 314.5 | |
| viscosity in Pa s | 54.7 | 43.65 | | 50.1 | |
| gelation time (min) | 2.18 | 2.18 | 7.5 | | |

### Example 2

### Analysis of Hydrogel

### Characteristics of the gel

The washed hydrogels will have typical properties outlined below.

**Table 5**

| Property | Low viscosity version | High viscosity version |
|---|---|---|
| Cross-linking density | 0,25% | 0,40% |
| Swelling rate | 80-120% | 50-70% |
| Rheol. elasticity modulus, G' | 10-80 Pa | 250-700 Pa |
| Rheol. Complex viscosity, η* | 2-10 Pas | 50-90 Pas |
| Dry matter | 1,6-3% | 3-5% |
| Refractive index | 1,335-1,338 | 1,338-1,340 |

### Example 3

Swelling occurs during wash procedure and will typically show a swelling profile as shown in below Figures 2 and 3.

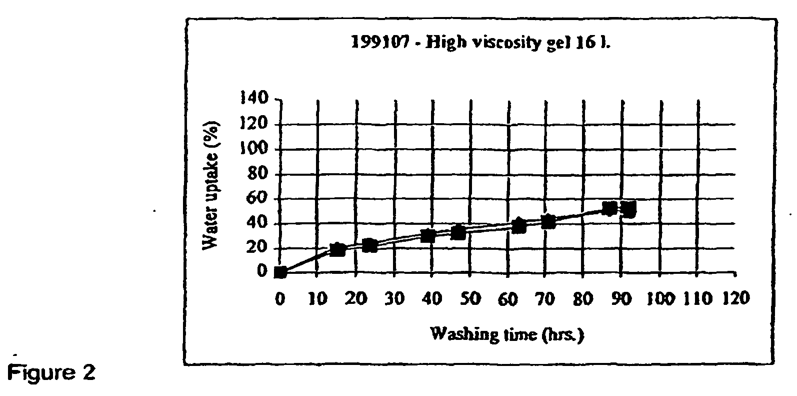

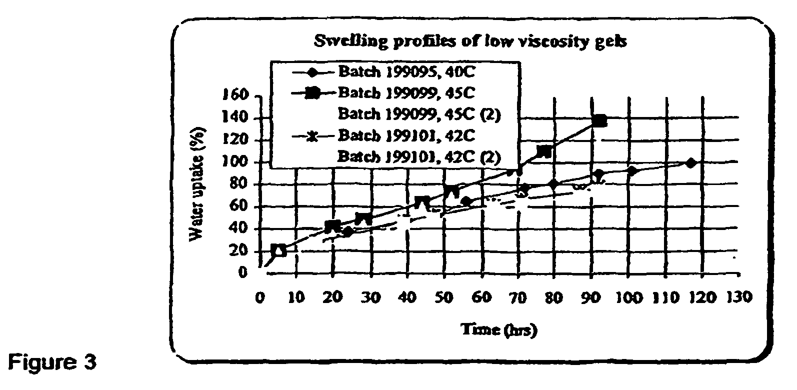

### Example 4

### Method of Administration and Clinical Results for Treating Incontinence

### Treatment by injection

The polyacrylamide hydrogel (solid weight content 2.5% and ca. 97.5% pyrogen-free water) is injected under the mucous membrane of the urethra so as to provide increased density of the urethra is carried out by during short procedure involving few complications.

The medical procedure involves injection of polyacrylamide gel under the mucous membrane of the urethra of women suffering from incontinence. Injection is via the external surface of the urethra and toward the submucosal membrane.
3 mL are injected at three depots sites are made along a single longitudinal position of the urethra. Depots 0.5 cm distally from the neck of the bladder were made.

The injections are carried out under a local anaesthetic, enabling the bladder to be filled subsequently and the patients to cough in order to establish immediately whether sufficient density for urinary incontinence has been achieved. On lack of effect, the injection may be repeated which is common in connection with treatment by injection.

Examination of the woman found the treatment by injection suitable according to the standards and regimes of the Gynaecology- Obstetrics department of Copenhagen's Amstsygehus (County Hospital) in Glostrup. Results and any complications are monitored at check-up examinations every three months for one year after the treatment.

## Claims

1. Use of a hydrogel comprising 0.5 to 25% by weight polyacrylamide, based on the total weight of the hydrogel for the preparation of a prosthetic device for the treatment or prevention of urinary incontinence.

2. Use of a hydrogel comprising 0.5 to 25% by weight polyacrylamide, based on the total weight of the hydrogel for the preparation of a prosthetic device for the treatment or prevention of anal incontinence.

3. Use of a hydrogel comprising at least 0.5% and less than 3.5% by weight polyacrylamide, based on the total weight of the hydrogel for the preparation of a prosthetic device for the treatment or prevention of vesicouretal reflux.

4. Use of a hydrogel obtainable by the steps comprising
combining acrylamide and methylene bis-acrylamide in amounts so as to give 0.5 to 25% by weight polymer, based on the total weight of the hydrogel;
radical initiation;
and washing with pyrogen-free water or saline solution;
for the preparation of a prosthetic device for the treatment or prevention of urinary incontinence.

5. Use of a hydrogel obtainable by the steps comprising
combining acrylamide and methylene bis-acrylamide In amounts so as to give 0.5 to 25% by weight polymer, based on the total weight of the hydrogel;
radical initiation;
and washing with pyrogen-free water or saline solution;
for the preparation of a prosthetic device for the treatment or prevention of anal incontinence.

6. Use of a hydrogel obtainable by the steps comprising
combining acrylamide and methylene bis-acrylamide in amounts so as to give at least 0.5% and less 3.5% by weight polymer, based on the total weight of the hydrogel; radical initiation;
and washing with pyrogen-free water or saline solution;
for the preparation of a prosthetic device for the treatment or prevention of vesicouretal reflux in a mammal.

7. The use according to any one of claims 1, 2, 4 and 5, wherein the hydrogel comprises less than 15% by weight polymer, based on the total weight of the hydrogel, preferably less 10%, more preferably less than 7.5%, even more preferably less than 5%, most preferably less than 3.5% by weight polymer, based on the total weight of the hydrogel.

8. The use according to any one of the preceding claims, wherein the hydrogel comprises at least 1% by weight polymer, based on the total weight of the hydrogel, preferably at least 1.5%, such as 1.6% by weight polyacrylamide, based on the total weight of the hydrogel.

9. The use according to any one of the preceding claims, wherein the hydrogel has a complex viscosity module of 2 to 50 Pa s, such as 2 to 40 Pa s, preferably 2 to 30 Pa s, more preferably 2 to 20 Pa s.

10. The use according to any one of the preceding claims, wherein the hydrogel has an elasticity modulus of 1 to 200 Pa, such as 2 to 175 Pa, typically 5 to 150 Pa, such as 10 to 100 Pa.

11. The use according to any one of the preceding claims, wherein said hydrogel comprises at least 75% by weight pyrogen-free water.

12. The use according to any one of claims 1 to 10, wherein the hydrogel comprises at least 80% by weight saline solution.

13. The use according to any one of the preceding claims, wherein the hydrogel further comprises cells, such as stem cells for cellular engraftment.

14. The use according to any one of the preceding claims, wherein the prosthetic device is administered by means of injection.

## Patentansprüche

1. Verwendung eines Hydrogels, umfassend 0,5 bis 25 Gew.-% Polyacrylamid, bezogen auf das Gesamtgewicht des. Hydrogels, zur Herstellung einer prothetischen Vorrichtung zur Behandlung oder Vorbeugung von Harninkontinenz.

2. Verwendung eines Hydrogels, umfassend 0,5 bis 25 Gew.-% Polyacrylamid, bezogen auf das Gesamtgewicht des Hydrogels, zur Herstellung einer prothetischen Vorrichtung zur Behandlung oder Vorbeugung von Stuhlinkontinenz.

3. Verwendung eines Hydrogels, umfassend mindestens 0,5 Gew.-% und weniger als 3,5 Gew.-% Polyacrylamid, bezogen auf das Gesamtgewicht des Hydrogels, zur Herstellung einer prothetischen Vorrichtung zur Behandlung oder Vorbeugung von vesikoureteralem Reflux.

4. Verwendung eines Hydrogels, erhältlich durch die Schritte, umfassend Kombinieren von Acrylamid und Methylen-bis-acrylamid in solchen Mengen, dass sich 0,5 bis 25 Gew.-% Polymer, bezogen auf das Gesamtgewicht des Hydrogels, ergeben;
Radikalinitiierung; und
Waschen mit pyrogenfreiem Wasser oder Salzlösung;
zur Herstellung einer prothetischen Vorrichtung zur Behandlung oder Vorbeugung von Harninkontinenz.

5. Verwendung eines Hydrogels, erhältlich durch die Schritte, umfassend Kombinieren von Acrylamid und Methylen-bis-acrylamid in solchen Mengen, dass sich 0,5 bis 25 Gew.-% Polymer, bezogen auf das Gesamtgewicht des Hydrogels, ergeben;
Radikalinitiierung; und
Waschen mit pyrogenfreiem Wasser oder Salzlösung;
zur Herstellung einer prothetischen Vorrichtung zur Behandlung oder Vorbeugung von Stuhlinkontinenz.

6. Verwendung eines Hydrogels, erhältlich durch die Schritte, umfassend Kombinieren von Acrylamid und Methylen-bis-acrylamid in solchen Mengen, dass sich mindestens 0,5 Gew.-% under weniger als 3,5 Gew.-% Polymer, bezogen auf das Gesamtgewicht des Hydrogels, ergeben;
Radikalinitiierung; und
Waschen mit pyrogenfreiem Wasser oder Salzlösung;
zur Herstellung einer prothetischen Vorrichtung zur Behandlung oder Vorbeugung von vesikoureteralem Reflux bei einem Säuger.

7. Verwendung nach einem der Ansprüche 1, 2, 4 und 5, wobei das Hydrogel weniger als 15 Gew.-% Polymer, bezogen auf das Gesamtgewicht des Hydrogels, vorzugsweise weniger als 10 Gew.-%, stärker bevorzugt weniger als 7,5 Gew.-%, noch stärker bevorzugt weniger als 5 Gew.-%, am stärksten bevorzugt weniger als 3,5 Gew.-% Polymer, bezogen auf das Gesamtgewicht des Hydrogels, umfasst.

8. Verwendung nach einem der vorstehenden Ansprüche, wobei das Hydrogel mindestens 1 Gew.-% Polymer, bezogen auf das Gesamtgewicht des Hydrogels, vorzugsweise mindestens 1,5 Gew.-%, wie z.B. 1,6 Gew.-% Polyacrylamid, bezogen auf das Gesamtgewicht des Hydrogels, umfasst.

9. Verwendung nach einem der vorstehenden Ansprüche, wobei das Hydrogel einen Komplexviskositätsmodul von 2 bis 50 Pa.s, wie z.B. 2 bis 40 Pa.s, vorzugsweise 2 bis 30 Pa.s, stärker bevorzugt 2 bis 20 Pa.s, aufweist.

10. Verwendung nach einem der vorstehenden Ansprüche, wobei das Hydrogel einen Elastizitätsmodul von 1 bis 200 Pa, wie z.B. 2 bis 175 Pa, typischerweise 5 bis 150 Pa, wie z.B. 10 bis 100 Pa, aufweist.

11. Verwendung nach einem der vorstehenden Ansprüche, wobei das Hydrogel mindestens 75 Gew.-% pyrogenfreies Wasser umfasst.

12. Verwendung nach einem der Ansprüche 1 bis 10, wobei das Hydrogel mindestens 80 Gew.-% Salzlösung umfasst.

13. Verwendung nach einem der vorstehenden Ansprüche, wobei das Hydrogel weiterhin Zellen, wie z.B. Stammzellen zur zellulären Verpflanzung, umfasst.

14. Verwendung nach einem der vorstehenden Ansprüche, wobei die prothetische Vorrichtung bei Injektion verabreicht wird.

## Revendications

1. Utilisation d'un hydrogel comprenant 0,5 à 25 % en poids de polyacrylamide, sur la base du poids total de l'hydrogel pour la préparation d'une prothèse pour le traitement ou la prévention de l'incontinence urinaire.

2. Utilisation d'un hydrogel comprenant 0,5 à 25 % en poids de polyacrylamide, sur la base du poids total de l'hydrogel pour la préparation d'une prothèse pour le traitement ou la prévention de l'incontinence anale.

3. Utilisation d'un hydrogel comprenant au moins 0,5 et moins de 3,5 % en poids de polyacrylamide, sur la base du poids total de l'hydrogel pour la préparation d'une prothèse pour le traitement ou la prévention du reflux vésicourétéral.

4. Utilisation d'un hydrogel que l'on peut obtenir au moyen des étapes comprenant une combinaison d'acrylamide et de méthylène bis-acrylamide en des quantités telles que cela donne 0,5 à 25 % en poids de polymère, sur la base du poids total de l'hydrogel ;
une initiation radicalaire ;
et un lavage avec de l'eau sans pyrogène ou une solution saline ;
pour la préparation d'une prothèse pour le traitement ou la prévention de l'incontinence urinaire.

5. Utilisation d'un hydrogel que l'on peut obtenir au moyen des étapes comprenant une combinaison d'acrylamide et de méthylène bis-acrylamide en des quantités telles que cela donne 0,5 à 25 % en poids de polymère, sur la base du poids total de l'hydrogel ;
une initiation radicalaire ;
et un lavage avec de l'eau sans pyrogène ou une solution saline ;
pour la préparation d'une prothèse pour le traitement ou la prévention de l'incontinence anale.

6. Utilisation d'un hydrogel que l'on peut obtenir au moyen des étapes comprenant une combinaison d'acrylamide et de méthylène bis-acrylamide en des quantités telles que cela donne au moins 0,5 et moins de 3,5 % en poids de polymère, sur la base du poids total de l'hydrogel ;
une initiation radicalaire ;
et un lavage avec de l'eau sans pyrogène ou une solution saline ;
pour la préparation d'une prothèse pour le traitement ou la prévention du reflux vésicourétéral chez un mammifère.

7. Utilisation selon l'une quelconque des revendications 1, 2, 4 et 5, dans laquelle l'hydrogel comprend moins de 15 % en poids de polymère, sur la base du poids total de l'hydrogel, de préférence moins de 10 %, voire moins de 7,5 %, voire même moins de 5 %, et plus préférentiellement moins de 3,5 % en poids de polymère, sur la base du poids total de l'hydrogel.

8. Utilisation selon l'une quelconque des revendications précédentes, dans laquelle l'hydrogel comprend au moins 1 % en poids de polymère, sur la base du poids total de l'hydrogel, de préférence au moins 1,5 %, tel que 1,6 % en poids de polyacrylamide, sur la base du poids total de l'hydrogel.

9. Utilisation selon l'une quelconque des revendications précédentes, dans laquelle l'hydrogel possède une viscosité de complexe de 2 à 50 Pa.s, telle que de 2 à 40 Pa.s, de préférence de 2 à 30 Pa.s, voire de 2 à 20 Pa.s.

10. Utilisation selon l'une quelconque des revendications précédentes, dans laquelle l'hydrogel possède un module d'élasticité de 1 à 200 Pa, tel que de 2 à 175 Pa, typiquement de 5 à 150 Pa, tel que de 10 à 100 Pa.

11. Utilisation selon l'une quelconque des revendications précédentes, dans laquelle ledit hydrogel comprend au moins 75 % en poids d'eau sans pyrogène.

12. Utilisation selon l'une quelconque des revendications 1 à 10, dans laquelle l'hydrogel comprend au moins 80 % en poids de solution saline.

13. Utilisation selon l'une quelconque des revendications précédentes, dans laquelle l'hydrogel comprend en outre des cellules, telles que des cellules souches pour greffe cellulaire.

14. Utilisation selon l'une quelconque des revendications précédentes, dans laquelle la prothèse est administrée par injection.
